**Europäisches Patentamt**

**European Patent Office**

**Office européen des brevets**

(19)

(11) Veröffentlichungsnummer : **0 355 391 B1**

# (12) EUROPÄISCHE PATENTSCHRIFT

(45) Veröffentlichungstag der Patentschrift :
**10.06.92 Patentblatt 92/24**

(51) Int. Cl.$^5$ : **A61F 15/00**, A61M 5/00,
A61B 19/02

(21) Anmeldenummer : **89113105.4**

(22) Anmeldetag : **18.07.89**

(54) **Behälter zur Abgabe trockener oder vorbefeuchteter Tücher, insbesondere mit Alkohol getränkter Tupfer, Verwandelbar in einenAbfallbehälter vorzugsweise für gebrauchte Kanülen.**

(30) Priorität : **19.07.88 DE 8809213 U**

(43) Veröffentlichungstag der Anmeldung :
**28.02.90 Patentblatt 90/09**

(45) Bekanntmachung des Hinweises auf die
Patenterteilung :
**10.06.92 Patentblatt 92/24**

(84) Benannte Vertragsstaaten :
**DE ES FR GB IT SE**

(56) Entgegenhaltungen :
**EP-A- 0 304 619**
**WO-A-89/04182**
**DE-A- 3 031 418**

(73) Patentinhaber : **HOLTSCH**
**Metallwarenherstellung Maria Holtsch**
**In den Faltern 13**
**W-6204 Taunusstein 4 (DE)**

(72) Erfinder : **Holtsch, Peter E.**
**In den Faltern 13**
**W-6204 Taunusstein (DE)**

EP 0 355 391 B1

## Beschreibung

Die Erfindung betrifft einen Behälter zur Abgabe trockener oder vorbefeuchteter Tücher, insbesondere mit Alkohol getränkter Tupfer, der durch einen Handgriff nach dem Entnehmen des letzten Tuches oder Tupfers in einen Abfallbehälter vorzugsweise für gebrauchte Einmalkanüten verwandelt wird.

Es ist eine Reihe von Spendern bekannt, denen man Tupfer oder dergleichen im trockenen oder angefeuchteten Zustand entnehmen kann. So ist in der Offenlegungsschrift DE 30 31 418 A1 ein Behälter beschrieben, in dem sich in einem auswechselbaren Magazinbeutel gefaltete Tupfer befinden, die mit dem Deckel verschraubt werden. Diese Erfindung hat den Nachteil, daß beim Auswechseln der Magazine eine Kontamination nicht zu vermeiden ist. Andere Spender bestehen meist aus einem Behälter, in dem Tupfer gestapelt oder aufgerollt und mit einem Deckel möglichst luftdicht verschlossen sind, der nur eine kleine, meist runde, Öffnung zur Entnahme der Tupfer oder dergleichen aufweist. Diese Behälter, die in der Herstellung retativ teuer sind, haben den Nachteil, daß sie nach dem Gebrauch nutzlos sind und weggeworfen werden. Ein Problem unserer Zeit ist die Anhäufung von Müll, der dadurch um weitere Hohlkörper bereichert wird.

Der als Spender für Tupfer oder dergleichen ausgebildete Behälter findet seinen Einsatz vor allem in Arztpraxen, in Krankenhäusern, Labors und Unfallstationen, also überall da, wo gleichzeitig Injektionen verabreicht werden und Blut entnommen wird. Eben dort fällt gefährlicher Abfall in Form von gebrauchten Nadeln und Kanülen der Einwegspritzen an.

Die Erfindung ermöglicht, die nutzlos gewordenen Hohlkörper der teeren Spender durch einen Handgriff in einen Abfallbehälter für gebrauchte Einmalkanülen, Nadeln, Spritzen oder dergleichen, zu verwandeln. Die Gebrauchszeit des Behälters wird dadurch verlängert, das Müllvolumen verringert.

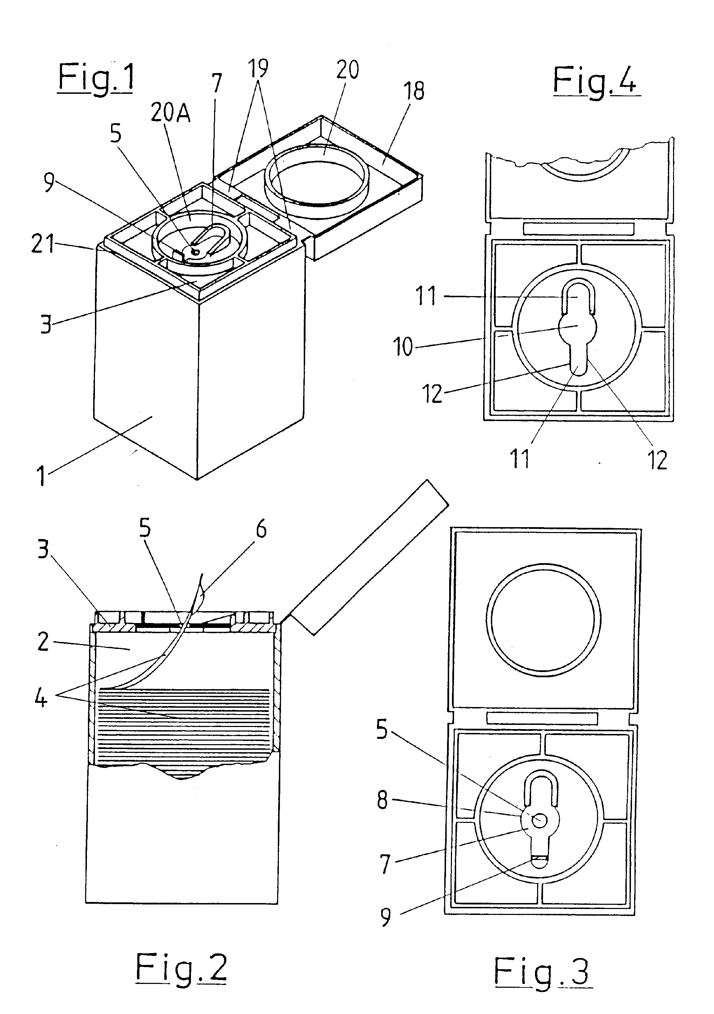

Anhand der Zeichnungen Fig. 1 bis Fig. 7 sott am Beispiel einer bevorzugten Ausführungsform des Behälters die Erfindung in verschiedenen Versionen näher erläutert werden.

Fig. 1 zeigt in perspektivischer Darstellung einen erfindungsgemäßen Behälter mit geöffnetem Deckel.

Fig. 2 zeigt eine Seitenansicht im Schnitt.

Fig. 3 zeigt eine Draufsicht vor dem Herausziehen der Tücher mit der Entnahmeöffnung.

Fig. 4 zeigt die Draufsicht auf die Einlaßöffnung nach dem Entfernen des Teiles mit der Entnahmeöffnung.

Fig. 5 zeigt eine andere Ausführungsform der Erfindung als Explosionszeichnung.

Fig. 6 zeigt eine perspektivische Darstellung des Behälters mit einer weiteren Ausführungsform.

Fig. 7 zeigt das Wandteil mit der Ein- und Auslaßöffnung von der Innenseite des Behälters.

Wie sich aus den Figuren der Zeichnungen ergibt, besteht der Behälter (1) aus mehreren Wänden, von denen eine Wand (3) die innere Kammer (2) abschließt, in der vorbefeuchtete oder trockene Tücher, Tupfer oder dergleichen lagern, die vorzugsweise aus aneinanderhängenden St reifen von Rechtecken bestehen, die über die Ecken diagonal miteinander verbunden sind. Die Tücher werden eines nach dem anderen aus dem Behälter gezogen. Aus diesem Grunde ist der Behälter mit einer Auslaßöffnung (5) versehen. Eine Ecke (6) vom Tuch, Tupfer od. dergleichen (3) ragt als Zipfel griffbereit aus der Auslaßöffnung (5) des Behälters. Die Auslaßöffnung (5) ist in einem zungen- oder schlüssellocharligen Wandteil (7) des Behälters angeordnet. Das zungen- oder schlüssellochähnliche Wandteil (7) wird durch eine rundum taufende Rinne (8), Perforation, Verzahnung od. dergleichen in einer Kontur abgegrenzt. Nach Entnahme des letzten Tupfers wird das abgegrenzte Wandstück mittels einer Zunge (9) herausgerissen oder einfach eingestoßen. Die so entstandene Einlaßöffnung (10) ist derartig ausgebildet, daß sie an den gegenüberliegenden Seiten in einen engeren und in einen weiteren Radius (11) mündet. Die gegenüberliegenden Wandränder (12) sind gabelschtüsselartig angeordnet und können zum Abschrauben der Kanülen von Einwegspritzen benützt werden, oder die Wandränder haben schräg angeordnete Materterhöhungen (13) zum Abstreifen von Einmalkanülen und Nadeln.

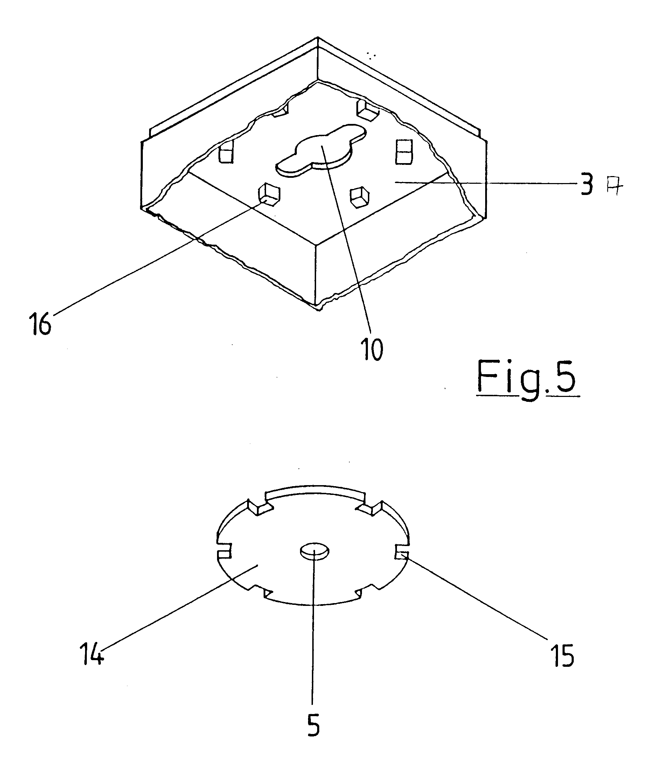

Eine andere Ausführungsform wird in Fig. 5 dargestellt. Bei dieser ist die Einlaßöffnung (10) in der beschriebenen Form bereits angeordnet und wird vorzugsweise von unten mit einem übertappenden Teil (14) abgedeckt. Das Teil (14) weist eine Auslaßöffnung (5) auf und hat beispielsweise an den Rändern Aussparungen (15), die in Materatanhäufungen (16) der Behälterwand (3) passen.

Nach Entnahme des letzten Tupfers wird das Teil (14) eingestoßen, so daß durch die Einlaßöffnung (10) in der beschriebenen Weise gebrauchte Kanülen, Nadeln, Spritzen od dergleichen abgeschraubt, abgestreift oder abgehebelt werden können.

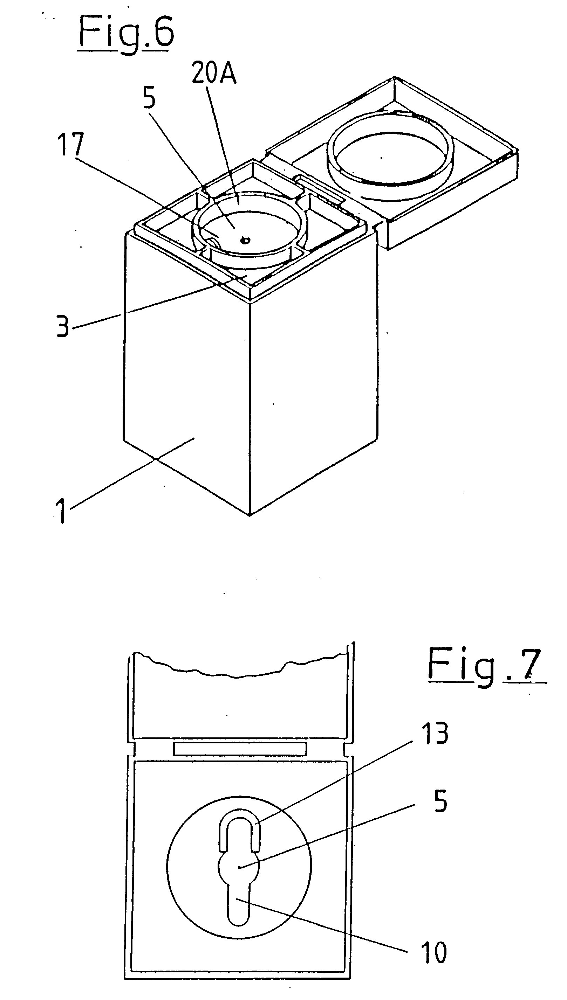

Bei einer weiteren Ausführungsform, dargestellt in Fig. 6 und 7, wird die Einlaßöffnung (10) durch eine selbstklebende Folie (17), die mit einer Auslaßöffnung (5) versehen ist, übertappend abgedeckt. Nach Entnahme des letzten Tupfers wird die Folie (17) entfernt, um die Einlaßöffnung zum Gebrauch, wie beschrieben, freizugeben. Bei dieser Ausführungsform sind die Wandränder mit den schräg angeordneten Materterhöhungen (13) der Einlaßöffnung nach innen angeordnet

Der Behätter (1) hat einen Deckel (18), der mit Scharnieren (19) verbunden ist. Im Deckel befindet sich ein Kragen (20), der in den Kragen (20a) im Wandstück (3), das die innere Kammer (2) abschließt, passend greift. Der Kragen (20a) ist um die Einlaßöffnung (10) und Auslaßöffnung (5) angeordnet. Das Wandstück (3) wird mit dem Behälter (1) durch eine Klebe- oder Schweißnaht (21) fest verbunden.

## Patentansprüche

1. Ein Spender für trockene oder vorbefeuchtete Tücher oder Tupfer, der aus einem Behälter mit einer Vielzahl von Wänden besteht, die einen Hohlraum für die Tücher oder Tupfer in gestapelter oder aufgerollter Form begrenzen, mit einer Öffnung (5) in einer der Wände zur Entnahme der Tücher oder Tupfer, dadurch gekennzeichnet, daß der Behälter mit einer oder mehreren materialgeschwächten Stelle(n) (8) in mindestens einer Wand oder mit mindestens einem Verschlußstück versehen ist, wobei das Verschlußstück eine Einlaßöffnung (10) überlappend oder passend abdeckt, daß die materialgeschwächten Stellen oder das Verschlußstück nach Entleerung des Behälters entfernt werden kann, so daß eine Einlaßöffnung (10) freigegeben wird, durch die gebrauchte medizinische Artikel in den Behälter befördert und dort gelagert werden können.

2. Behälter nach Anspruch 1 dadurch gekennzeichnet, daß die geschwächte Stelle als Rinne oder periodische Unterbrechung ausgebildet ist.

3. Behälter nach Anspruch 1 und 2 dadurch gekennzeichnet, daß das durch Schwächung eingegrenzte Wandstück mittels einer Zunge, Lasche oder dergleichen herausgezogen werden kann.

4. Behälter nach Anspruch 1 bis 3 dadurch gekennzeichnet, daß die materialgeschwächte(n) Stelle(n) nur um die Entnahmeöffnung herum angeordnet ist/sind.

5. Behälter nach Anspruch 1 bis 4 dadurch gekennzeichnet, daß die Verschlußstücke mit einer Entnahmeöffnung versehen sind.

6. Behälter nach Anspruch 1 bis 5 dadurch gekennzeichnet, daß die Verschlußstücke durch Materialaussparungen, Materialerhöhungen oder Verzahnungen an der Wand gehalten sind.

7. Behälter nach Anspruch 1 bis 6 dadurch gekennzeichnet, daß die Verschlußstücke an der Innenseite (3) des Behälters angeordnet sind.

8. Behälter nach Anspruch 1 bis 7 dadurch gekennzeichnet, daß das/die Verschlußstück(e) (14) und die entsprechende Behälterwand so ausgeformt sind (16), daß das Verschluffstück paßgenau eingesetzt werden kann.

9. Behälter nach Anspruch 1 bis 8 dadurch gekennzeichnet, daß an den Rändern der Einlaßöffnungen (10) Kanten in Form von Schrägen (7) angebracht sind.

10. Behälter nach Anspruch 1 bis 9 dadurch gekennzeichnet, daß die Wand mit der Einlaßöffnung (10) oberhalb oder unterhalb überlappend mit einem Einsatz oder zusätzlichen Wandstücken versehen ist, die eine sich von der Einlaßöffnung (10) unterscheidende Entnahmeöffnung (5) haben.

11. Behälter nach Anspruch 1 bis 10 dadurch gekennzeichnet, daß eine Entnahmeöffnung (5) im Einsatz (14) oder Wandstück angebracht ist, die sich nach Entfernen des Einsatzes oder Wandstückes verändert, vorzugsweise vergrößert und zur Einlaßöffnung (10) wird.

12. Behälter nach Anspruch 1 bis 11 dadurch gekennzeichnet, daß die Einlaßöffnung (10) durch parallel verlaufende Kanten (12) gebildet wird, die sich an einer oder mehreren beliebigen Positionen erweitern und Radien bilden.

13. Behälter nach Anspruch 1 bis 12 dadurch gekennzeichnet, daß um die Entnahme- oder Einlaßöffnung ineinanderpassende Kragen (20, 20A) zum Verschluß ausgebildet sind.

14. Behälter nach Anspruch 1 bis 13 dadurch gekennzeichnet, daß der allseitig verschlossene Behälter Schweiß- (21) oder Klebenähte aufweist.

## Claims

1. A dispenser for dry or pre-moistened tissues or wipes consisting of a container with a plurality of walls which define a cavity for the tissues or wipes in stacked or rolled up form, having an opening (5) in one of the walls for removing the tissues or wipes, characterised in that the container is provided with one or more frangible area(s) (8) in the material of at least one wall or is provided with at least one closure member which closes off an inlet opening (10) by overlapping or fitting into it, and in that, after the container has been emptied, the frangible area(s) or the closure member can be removed so that an inlet opening (10) is exposed through which used medical articles can be placed in the container and stored therein.

2. A container according to claim 1, characterised in that the frangible area takes the form of a channel or intermittent perforations.

3. A container according to claims 1 and 2, characterised in that the section of wall defined by the frangible area can be pulled out by means of a tongue, flap or the like.

4. A container according to claims 1 to 3, characterised in that the frangible area(s) is/are arranged only around the removal opening.

5. A container according to claims 1 to 4, characterised in that the closure members are provided with a removal opening.

6. A container according to claims 1 to 5, characterised in that the closure members are held on the wall by recesses, elevations or teeth formed in the material.

7. A container according to claims 1 to 6, characterised in that the closure members are arranged on the inside (3) of the container.

8. A container according to claims 1 to 7, characterised in that the closure member(s) (14) and the corresponding container wall are of a configuration (16) such that the closure member can be inserted to fit exactly.

9. A container according to claims 1 to 8, characterised in that edges in the form of slants (7) are provided around the margins of the inlet openings (10).

10. A container according to claims 1 to 9, characterised in that the wall containing the inlet opening (10) is provided with an insert or additional wall portions overlapping therewith, either above or below, the insert or additional wall portions having a removal opening (5) distinct from the inlet opening (10).

11. A container according to claims 1 to 10, characterised in that a removal opening (5) is provided in the insert (14) or wall portion, which changes, preferably becomes larger, after removal of the insert or wall portion and becomes the inlet opening (10).

12. A container according to claims 1 to 11, characterised in that the inlet opening (10) is formed by parallel edges (12) which widen out at one or more desired positions and form radii.

13. A container according to claims 1 to 12, characterised in that collars (20, 20A) interlocking with each other around the removal or inlet opening are constructed to form the closure.

14. A container according to claims 1 to 13, characterised in that the container which is sealed on all sides has welded seams (21) or glued seams.

## Revendications

1. Distributeur de serviettes ou tampons secs ou préhumidifiés, constitué d'un récipient ayant une multiplicité de parois qui délimitent une chambre de réception des serviettes ou des tampons à l'état empilé ou enroulé, avec une ouverture (5) ménagée dans l'une des parois et permettant de retirer les serviettes ou tampons, caractérisé en ce que le récipient est muni, dans au moins une paroi ou dans au moins une pièce de fermeture, d'un ou de plusieurs emplacement(s) (8) où la matière est affaiblie, la pièce de fermeture recouvrant, avec dépassement ou de manière ajustée, une ouverture d'entrée (10), en ce que les emplacements où la matière est affaiblie ou la pièce de fermeture peuvent être enlevés après que le récipient a été vidé, de manière à dégager l'ouverture d'entrée (10) par laquelle les objets médicaux ayant servi peuvent être transportés dans le récipient et y être entreposés.

2. Récipient suivant la revendication 1, caractérisé en ce que l'emplacement affaibli est constitué sous la forme d'une rainure ou d'une partie interrompue périodiquement.

3. Récipient suivant la revendication 1 ou 2, caractérisé en ce que la partie de paroi délimitée par l'affaiblissement peut être enlevée au moyen d'une languette, d'une patte ou analogue.

4. Récipient suivant l'une des revendications 1 à 3, caractérisé en ce que le(s) emplacement(s) où la matière est affaiblie n'est-ne sont disposés qu'autour de l'ouverture de retrait.

5. Récipient suivant l'une des revendications 1 à 4, caractérisé en ce que les pièces de fermeture sont munies d'une ouverture de retrait.

6. Récipient suivant l'une des revendications 1 à 5, caractérisé en ce que les pièces de fermeture sont maintenues sur la paroi par des évidements de matière, des surélévations de matière ou par des dentures.

7. Récipient suivant l'une des revendications 1 à 6, caractérisé en ce que les pièces de fermeture sont disposées sur la face intérieure (3) du récipient.

8. Récipient suivant l'une des revendications 1 à 7, caractérisé en ce que la/les pièce(s) de fermeture (14) et la paroi correspondante du récipient sont conformées (16) de manière à pouvoir insérer la pièce de fermeture à ajustement précis.

9. Récipient suivant l'une des revendications 1 à 8, caractérisé en ce que, sur les bords des ouvertures d'entrée (10), sont ménagées des arêtes en forme de parties inclinées (7).

10. Récipient suivant l'une des revendications 1 à 9, caractérisé en ce que la paroi ayant l'ouverture d'entrée (10) est munie au-dessus et au-dessous et avec recouvrement d'une pièce rapportée ou de parties supplémentaires de paroi, qui ont une ouverture de retrait (5) distincte de l'ouverture d'entrée (10).

11. Récipient suivant l'une des revendications 1 à 10, caractérisé en ce qu'il est prévu une ouverture de retrait (5) dans la pièce rapportée (14) ou dans la partie de paroi, qui se modifie après l'enlèvement de la pièce rapportée ou de la partie de paroi, de préférence en s'agrandissant et en devenant une ouverture d'entrée (10).

12. Récipient suivant l'une des revendications 1 à 11, caractérisé en ce que l'ouverture d'entrée (10) est formée d'arêtes (12) s'étendant parallèlement, qui s'élargissent en une ou en plusieurs positions et qui forment des rayons.

13. Récipient suivant l'une des revendications 1 à 12, caractérisé en ce qu'autour de l'ouverture de retrait ou d'entrée, sont constitués, pour la fermeture, des collets (20, 20A) qui s'emboîtent l'un dans l'autre.

14. Récipient suivant l'une des revendications 1 à 13, caractérisé en ce que le récipient fermé de tous côtés comporte des cordons de soudure (21) ou de collage.

Fig.1

Fig.4

Fig.2

Fig.3

3 A

16

10

# Fig.5

14

15

5

Fig.6

Fig.7